(19) Europäisches Patentamt
European Patent Office
Office européen des brevets

(11) Publication number: 0 070 686 B1

(12) EUROPEAN PATENT SPECIFICATION

(45) Date of publication of patent specification: 02.10.85

(51) Int. Cl.$^4$: G 01 N 33/542

(21) Application number: 82303700.7

(22) Date of filing: 14.07.82

# (54) Non-radiative energy transfer immunochemical technique.

(30) Priority: 17.07.81 US 284400

(43) Date of publication of application: 26.01.83 Bulletin 83/04

(45) Publication of the grant of the patent: 02.10.85 Bulletin 85/40

(84) Designated Contracting States: BE CH DE FR GB IT LI NL

(58) References cited:
EP-A-0 032 286
GB-A-2 008 247
GB-A-2 018 424
GB-A-2 018 986

(73) Proprietor: AMOCO CORPORATION
200 East Randolph Drive
Chicago Illinois 60601 (US)

(72) Inventor: Morrison, Larry Edward
4913 Spencer
Lisle Illinois 60532 (US)
Inventor: Heller, Michael James
30 W 057-104 Granada
Naperville Illinois 60540 (US)

(74) Representative: Ritter, Stephen David et al
Mathys & Squire 10 Fleet Street
London EC4Y 1AY (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 070 686 B1

**Description**

Background of the invention

Immunochemical type assays are rapidly becoming a major segment of the clinical diagnostic market. Presently, nephelometric and radio-immunoassays (RIA) represent the most well established techniques in immuno-diagnostics. New techniques such as the enzyme multiplied immunoassay technique (EMIT)®, enzyme immunoassay (EIA), and heterogeneous fluorescent immunoassays have been introduced and have met with varying degrees of success. Most of these assay techniques are aimed at replacing RIA's because of the numerous problems involved in handling radioisotopes.

For example, U.S. 4,104,029 to Maier, Jr. teaches a chemiluminescent assay method wherein a competition reaction is set up between chemiluminescent labeled antigens and sample antigens. These two species compete for the limited binding sites available provided by the antibodies present in the assay. The resulting luminescence is inversely related to the amount of antigen in the sample.

Other methods have used the concept of non-radiative energy transfer, which occurs when a light emitting species is in close proximity to a light absorbing species whose absorbance spectrum overlaps the emission spectrum of the emitting species. Under favorable conditions, all of the light energy which would otherwise be released by the emitter in the form of light is instead transferred to the absorbing species. This condition is described by the Forster equations (see "Energy Transfer and Organic Photochemistry", *Technique of Organic Chemistry*, Vol. XIV eds. P. A Leermakers and A. Weissberger, pp. 17—132 (1969), Interscience, New York) which suggest that the rate of energy transfer is inversely proportional to the sixth power of the distance between the absorbing and emitting species. Hence it is necessary that the emitting and absorbing species be very close together to effect energy transfer.

For example, U.S. 3,996,345 to Ullman et al. teaches a method for carrying out immunoassays employing antibodies and a fluorescer-quencher pair. Upon irradiation, the resulting fluorescence is related to the amount of the component of interest present. A number of U.S. patents by Ullman or Ullman et al. have issued which are closely related in subject matter include 3,998,943; 4,160,016; 4,174,384; 4,161,515; and 4,199,559. All of these patents relate to the concept of fluorescent quenching in some manner. Similar methods involving fluorescent quenching are described in two articles. (See "Fluorescent Excitation Transfer Immunoassay," *The Journal of Biological Chemistry*, Vol. 251, No. 14, pp. 4172—4178 (July 25, 1976) and "Fluorescamine and Fluorescein as Labels in Energy-tranfer Immunoassay," *Analyst*, Vol. 105, pp. 91—92 (1980)).

It has now been discovered that the specificity of antibody-antigen reactions can provide a means for bringing a chemiluminescent species and a light absorbing and re-emitting species in sufficiently close proximity to satisfy the energy transfer requirements. This eliminates the need for an external source of exciting light and can result in a higher degree of sensitivity.

According to one aspect of the present invention there is provided a method for assaying antigens having multiple binding sites, comprising:

(a) introducing a sample into a reagent solution comprising (i) an absorber/emitter conjugated antibody, (ii) a chemiluminescence catalyst conjugated antibody and (iii) a chemiluminescence reagent comprising a chemiluminescence cofactor generator catalyst conjugated antibody capable of producing a chemiluminescence cofactor which can induce a light response in the presence of the chemiluminescence catalyst; and

(b) detecting any light response emitted by the absorber/emitter conjugated antibody.

The invention also provides a reagent kit for carrying out the above method comprising components (i) to (iii) set forth above.

For purposes herein, the term "absorber/emitter" refers to any species which absorbs light energy and emits light of a different wavelength. This term particularly includes fluorophores and phosphores. Also, the terms "chemiluminescent" or "chemiluminescence" include the closely related phenomenon of bioluminescence. The term "antibody" shall include antibody analogs.

Because of the close proximity of the chemiluminescence catalyst conjugated antibody and the absorber/emitter conjugated antibody when bound to a single antigen, a highly efficient energy transfer occurs from the chemiluminescence catalyst to the absorber/emitter. The absorber/emitter dissipates the energy by emitting light of a longer wavelength than the chemiluminescent light absorbed. Hence, in the case of a fluorophore absorber/emitter, fluorescent light would only be substantially emitted when antigen was present in the sample. Any fluorescent light detected from the system would be related to the amount of antigen in the sample when light-labeled reagent antibodies were present in nonlimiting amounts. The amount of fluorescent light emitted from the reagent solution as background light would be negligible because it is necessary that all of the appropriate reagents concurrently be very close together. In solution the average distance between the chemiluminescence catalyst and the fluorophore would be such that negligible energy transfer would occur without the presence of antigen, which provides the binding sites (surface antigens) for the antibody conjugated reagents. These binding sites are sufficiently close together to permit adequate energy transfer.

As indicated, it is a feature of this invention to include among the chemiluminescent reagents a "chelimuninescence cofactor generator catalyst" conjugated antibody which catalyzes the formation of a chemiluminescence cofactor. For purposes of this specification, a "cofactor" is any reactant species

necessary to produce a light-emitting reaction, excluding the catalyst discussed herein. Since both catalysts have been conjugated to an antibody which binds them to the antigen in close proximity, the likelihood of a chemiluminescent light response sufficient to excite the absorber/emitter is increased by the local generation of a necessary cofactor in the light-producing reaction.

It is also preferable to include among the chemiluminescence reagents a "chemiluminescence cofactor scavenger" catalyst. This catalyst remains free in solution and is not conjugated to an antibody. It serves to eliminate any particular cofactor which may be present in the reagent solution generally, since it is desirable to only have the necessary cofactors and other reagents present in the vicinity of the antigen-bound reagents. Hence, the presence of such a cofactor scavenger catalyst aids in reducing background light emissions by eliminating a key species in the light-producing reaction. On the other hand, the presence of the scavenger catalyst in the solution generally will not affect the assay since the concentration of the cofactor near the antigen is too high for the cofactor scavenger catalyst to have an appreciable effect. Hence, the desired positive reaction still occurs.

## Brief description of the drawing

Figure 1 illustrates one example of this invention using a specific reagent system in the presence of sample antigen.

Figure 2 illustrates the same system as in Figure 1, but without the presence of sample antigen.

## Detailed discussion of the invention

In carrying out the methods of this invention, the highly specific antibody-antigen reactions are used to bring together, in very close proximity, an excitable fluorescent or phosphorescent light source and an exciting chemiluminescent light source. The purpose of utilizing this principle is to achieve a highly sensitive and reliable method for assaying antigens in a given sample. The antigen-antibody affinity provides a means for accomplishing this purpose by affording a unique way of bringing together certain necessary reactants which emit a characteristic light response when sample antigen is present.

Directing attention to Figure 1, the invention can be more easily understood by examining a specific representative example. Figure 1 illustrates the various reactions which take place when an antigen is present in the reagent solution as a result of combining the reagent solution with the sample to be assayed. The antigen contains a plurality of binding sites which provide places for the various reagents to attach themselves. Shown are a fluorophore conjugated antibody (absorber/emitter conjugated antibody), a peroxidase conjugated antibody (chemiluminescence catalyst conjugated antibody), and a glucose oxidase conjugated antibody (chemiluminescence cofactor generator catalyst conjugated antibody), all necessarily bound and concentrated in a small space relative to the balance of the reagent solution. This close proximity of reactant species creates a much greater likelihood of a fluorescent light-emitting reaction. The pertinent chemiluminescent reactions for this particular reagent system are as follows:

$$\text{(1)}\quad \text{Glucose}+O_2 \xrightarrow[\text{Oxidase}]{\text{Glucose}} H_2O_2+\text{Gluconate}$$

$$\text{(2)}\quad H_2O_2+\text{Luminol} \xrightarrow{\text{Peroxidase}} \text{Oxyluminol}+H_2O+N_2+hv$$

As can be seen from reaction (2), both hydrogen peroxide ($H_2O_2$) and luminol must contact the peroxidase catalyst in order for a light-emitting reaction to occur. To this end it is possible (and within the scope of this invention) to simply have sufficient amounts of both cofactors (hydrogen peroxide and luminol) in the reagent solution, but such a situation would lead to considerable background chemiluminescent light being emitted regardless of whether or not antigen is present. This background light could be filtered, but such a situation is not preferable. To avoid an undue amount of background emissions, a chemiluminescent cofactor generator catalyst conjugated antibody (a glucose oxidase conjugated antibody) is included in the reagent solution. This goes one step further in assuring that chemiluminescence will most likely occur only if antigen is present, since one of the necessary cofactors ($H_2O_2$) is only produced in the vicinity of the antigen-bound peroxidase catalyst. As further insurance against unwanted, chance chemiluminescent emissions occurring, the reagent solution illustrated in Figure 1 contains a chemiluminescence cofactor scavenger catalyst (catalase) which removes hydrogen peroxide from the reagent solution generally according to the following reaction:

$$\text{(3)}\quad 2H_2O_2 \xrightarrow{\text{catalase}} 2H_2O+O_2$$

Hence, Figure 1 illustrates how glucose oxidase, peroxidase, and a fluorophore are all closely held in the vicinity of each other by being bound to the antigen with conjugated antibodies. Glucose and oxygen in the reagent solution contact the glucose oxidase and form hydrogen peroxide. The hydrogen peroxide, being generated close to the peroxidase catalyst, combines with luminol present in the reagent solution to

emit chemiluminescent light energy (λ≤500 nm). However, because of the close proximity of the peroxidase and the fluorophore, which is also closely bound to the antigen, this emitted light energy is absorbed and converted to fluorescent light by the fluorophore (λ≥500 nm). Any chemiluminescent light is filtered, allowing the detector only to detect the fluorescent light, which is proportional to the amount of antigen present in the sample.

Figure 2 illustrates the same reagent system of Figure 1, but without antigen present in the sample. Because all the reagents necessary to produce the fluorescent light response are randomly dispersed in solution, the likelihood of chemiluminescence occurring is small, particularly so because of the presence of catalase to scavenge any hydrogen peroxide formed. The chances of a secondary light emission (fluorescence) occurring is even smaller, since it is necessary that the excited chemiluminescent emitter be formed very close to the fluorophore. Otherwise there will be insufficient energy transfer to accomplish a detectable fluorescent response.

The necessary antibodies to be used for preparing reagents can be obtained by known methods, such as isolation of antibodies from the blood of an animal previously injected with the antigen of interest (See Herman N. Eisen, *Immunology*, Ch. 15 and 17, Harper Row, New York, 1974) or fusing an antibody producing cell with a myeloma cell to produce a stable producer of monoclonal antibodies (See R. H. Kennet and T. J. McKearn (eds.) *Monoclonal Antibodies*, Plenum Press, New York, 1981). Both references are hereby incorporated by reference.

Suitable chemiluminescence catalysts include peroxidase, bacterial luciferase, and firefly luciferase, which produce light according to the following separate reactions:

$$(4)\quad H_2O_2+\text{Luminol}\xrightarrow{\text{Peroxidase}}\text{Oxyluminol}+H_2O+N_2+hv$$

$$(5)\quad FMNH_2+O_2+RCHO\xrightarrow{\text{Bacterial Luciferase}}FMN+RCOOH+H_2+hv$$

$$(6)\quad \text{Luciferin}+ATP+O_2\xrightarrow{\text{Firefly Luciferase}}\text{Oxyluciferin}+AMP+CO_2+PPi+hv$$

wherein $FMNH_2$ is reduced flavin mononucleotide, R is straight carbon chain having about 8—12 carbons, FMN is flavin mononucleotide, ATP is adenosine triphosphate, AMP is adenosine monophosphate, and PPi is inorganic phosphates.

In choosing the particular absorber/emitter for a given reagent system, it is necessary that it possess absorbance in the spectral region of the chemiluminescence produced by the chemiluminescence reagents. It is preferable that the emission of the absorber/emitter be of a long enough wavelength to be effectively distinguished from the chemiluminescence emitted by the reagent system. For example, two chemiluminescent reactions of primary interest are luminol oxidation by hydrogen peroxide and aldehyde oxygenation (e.g. isobutyraldehyde and propanal). Both of these reactions are catalyzed by peroxidase. Suitable absorber/emitters for the luminol chemiluminescent reaction include free base porphyrins such as uroporphyrin and tetracarboxyphenylporphyrin, metalloporphyrins containing such metals as magnesium or zinc, tetraphenylcarboxyporphyrins, perylene, anthracene, 7-methyldibenzo (a,h) pyrene, and other polycyclic aromatics having conjugated ring systems of sufficient size to produce strong absorbance in the region of luminol chemiluminescence (between 400 and 450 nm). The absorber/emitters may be easily sulfonated and activated for conjugation with antibodies by formation of the sulfonic acid chlorides by general synthetic procedures. Also, carboxylation may be performed if required and acid chlorides formed of these to activate for coupling to antibodies.

Suitable absorber/emitters for the chemiluminescence resulting from aldehyde oxygenation include the above-mentioned porphyrins and polynuclear aromatics. However, halogenation of the polynuclear aromatics is required in order to provide efficient transfer of energy from the chemiluminescent emitter since it emits from a triplet excited state. Examples of appropriate halogenated polynuclear aromatics are 9,10-dibromoanthracene, 9,10-dibromo-2,6-anthracene disulfonic acid, 3,10-dibromo-4,9-perylene-dicarboxylate, and 3,9- or 3,10-dibromoperylene. If required, sulfonation or carboxylation as described are also easily performed on these compounds by general synthetic procedures.

Phosphorescent compounds may also be used as absorber/emitters in the chemiluminescent aldehyde system due to the triplet nature of the emitter. In general, however, phosphorescent absorber/emitters are not preferable due to their sensitivity toward quenching in the presence of oxygen. This class of compounds includes α-diketones such as 2,3-butadione, 1-[carboxyphenyl]-1,2-pentanedione, and 1-phenyl-1,2-propanedione. In the latter case coupling to antibody or antigen is afforded through substitution of the phenyl ring with sulfonic acid chlorides, acid chlorides, or diazo activated intermediates.

The methods of attaching the absorber/emitters, chemiluminescence catalysts, and chemiluminescence cofactor generator catalysts to the appropriate antibodies will depend upon the particular species concerned. For example, the sulfonic acid derivative of a fluorophore such as 9,10-dibromo-2-anthracene sulfonic acid may be converted to the sulfonic acid chloride by treatment with $POCl_3$ in dichloroethane. The sulfonic acid chloride will then couple to the ε-amino groups of lysine

residues of antibodies or N-terminal amino acids in aqueous solution. The carboxylic acid derivative of a fluorophore such as dibromoperylene dicarboxylic acid may be converted to the acid chloride by treatment with thionyl chloride. The acid chloride will couple to antibody in the same manner as the sulfonic acid chlorides. These and other common coupling procedures are known in the literature (e.g. see *Immobilized Enzymes, Antigens, Antibodies, and Peptides*, H. H. Weetall (ed.), Ch. 1, Mareel Dekker, New York (1975) herein incorporated by reference). Many peroxidase conjugates of antibodies are commercially available and are prepared by oxidation of carbohydrate residues on peroxidase to form an aldehyde through which amino groups of antibodies are reacted forming Schiff base linkages. These Schiff bases are further stabilized by reduction with borohydride. Conjugation of glucose oxidase with an antibody has been reported using the bifunctional reagent succinimidyl 4-(N-maleimidomethyl)cyclohexane-1-carboxylate (S. Yoshitake *et al.*, *European Journal of Biochemistry*, Vol. 101, 395—399, herein incorporated by reference).

It will be appreciated that the chemiluminescence reagents or cofactors necessary for inducing a light response in the presence of the selected chemiluminescence catalyst conjugated antibody are well documented and can easily be determined by those skilled in biochemistry. For example, in the presence of a peroxidase chemiluminescence catalyst, the light-inducing reagents or cofactors could include either (1) luminol and hydrogen peroxide or (2) an aldehyde (such as isobutyraldehyde or propionaldehyde), dissolved oxygen, and ethanol and/or sodium pyrophosphate and/or sodium phosphate. In the presence of a firefly luciferase catalyst, the light-inducing reagents would include the combination of luciferin, ATP, $Mg^{++}$ ion, and dissolved oxygen. In the presence of a bacterial luciferase catalyst, the light-inducing reagents would include $FMNH_2$, a long chain aldehyde, and dissolved oxygen. (See reaction equations (4), (5) and (6) previously mentioned.)

The assay can be carried out in any suitable container having a transparent wall through which the light emissions can be detected. The assay is preferably conducted at room temperature for convenience. In general, the concentrations of the various reagents in the reagent solution will be about $10^{-8}$ to $10^{-6}$M for antibody conjugates; $10^{-3}$M for luminol when used; $5\times10^{-3}$M for hydrogen peroxide where used; $10^{-3}$ to $10^{-2}$M for aldehydes where used; $10^{-2}$ to $10^{-1}$M for pyrophosphate where used; and $10^{-2}$ to $10^{-1}$M phosphate where applicable.

One example of a reagent solution for assaying a blood or urine sample could include peroxidase conjugated antibodies ($\sim10^{-8}$M), carboxy-phenylporphyrin conjugated antibodies ($\sim10^{-8}$M), luminol ($\sim10^{-3}$M), and hydrogen peroxide ($\sim5\times10^{-3}$M). The reagent solution should also include glucose oxidase conjugated antibodies ($\sim10^{-8}$M), glucose ($\sim10^{-3}$M), and catalase ($\sim10^{-6}$M).

Another specific reagent solution would include peroxidase conjugated antibodies ($\sim10^{-6}$M), 3,10-dibromoperylene conjugated antibodies ($\sim10^{-6}$M), isobutyraldehyde ($\sim5\times10^{-2}$M), dissolved oxygen, ethanol, sodium phosphate, sodium pyrophosphate, and a suitable chemiluminescence cofactor generator catalyst conjugated antibody.

A further specific reagent solution would include firefly luciferase conjugated antibodies ($\sim10^{-8}$M), uroporphyrin conjugated antibodies ($\sim10^{-7}$ g/ml.), ATP ($\sim10^{-6}$M), luciferin ($\sim5\times10^{-5}$M), $Mg^{+2}$ ion ($\sim10^{-2}$M), dissolved oxygen and a suitable chemiluminescence cofactor generator catalyst conjugated antibody.

**Claims**

1. A method for assaying antigens having multiple binding sites, comprising:

(a) introducing a sample into a reagent solution comprising (i) an absorber/emitter conjugated antibody, (ii) a chemiluminescence catalyst conjugated antibody and (iii) a chemiluminescence reagent comprising a chemiluminescence cofactor generator catalyst conjugated antibody capable of producing a chemiluminescence cofactor which can induce a light response in the presence of the chemiluminescence catalyst; and

(b) detecting any light response emitted by the absorber/emitter conjugated antibody.

2. A method according to Claim 1 wherein the reagent comprises a chemiluminescence cofactor scavenger catalyst.

3. A method according to Claim 1 or Claim 2 wherein the absorber/emitter is a fluorophore.

4. A method according to Claim 3 wherein the fluorophore is selected from uroporphyrin, tetracarboxyphenylporphyrin, perylene, anthracene, 7-methyldibenzo(a,h)pyrene, 9,10-dibromoanthracene, riboflavin, 3,9-dibromoperylene, 3,10-dibromoperylene, and 3,10-dibromo-4,9-perylenedicarboxylate.

5. A method according to Claim 1 or Claim 2 wherein the absorber/emitter is a phosphore.

6. A method according to Claim 5 wherein the phosphore is selected from 2,3-butadione, 1-[carboxyphenyl]-1,2-pentanedione and 1-phenyl-1,2-propanedione.

7. A method according to any preceding claim wherein the chemiluminescence catalyst is selected from peroxidase, bacterial luciferase and firefly luciferase.

8. A kit for preparing a reagent solution for assaying large antigens by the method of Claim 1 comprising (i) an absorber/emitter conjugated antibody, (ii) a chemiluminescence catalyst conjugated antibody and (iii) a chemiluminescence reagent comprising a chemiluminescence cofactor generator

catalyst conjugated antibody capable of producing a chemiluminescence cofactor which can induce a light response in the presence of the chemiluminescence catalyst.

9. A kit according to Claim 8 comprising a chemiluminescence cofactor scavenger catalyst.

10. A kit according to Claim 9 comprising peroxidase conjugated antibodies, riboflavin conjugated antibodies, luminol, glucose oxidase conjugated antibodies, glucose and catalase.

**Patentansprüche**

1. Verfahren zur Prüfung von Antigenen, die mehrfache Bindungsstellen haben, dadurch gekennzeichnet, daß man

(a) eine Probe in eine Reagenzlösung einführt, die (i) einen mit einem Absorber/Emitter verbundenen Antikörper, (ii) einen mit einem Chemilumineszenzkatalysator verbundenen Antikörper und (iii) ein Chemilumineszenzreagenz enthält, das aus einem mit einem Chemilumineszenzcofaktorgeneratorkatalysator verbundenen Antikörper besteht, der zur Bildung eines Chemilumineszenzcofaktors befähigt ist, welcher in Gegenwart des Chemilumineszenzkatalysators ein Lichtsignal hervorrufen kann, und

(b) das von dem mit einem Absorber/Emitter verbundenen Antikörper emittierte Lichtsignal ermittelt.

2. Verfahren nach Anspruch 1, dadurch gekennzeichnet, daß das Reagenz aus einem Chemilumineszenzcofaktorfängerkatalysator besteht.

3. Verfahren nach Anspruch 1 oder Anspruch 2, dadurch gekennzeichnet, daß der Absorber/Emitter ein Fluorophor ist.

4. Verfahren nach Anspruch 3, dadurch gekennzeichnet, daß das Fluorophor ausgewählt ist aus Uroporphyrin, Tetracarboxyphenylporphyrin, Perylen, Anthracen, 7-Methyldibenzo(a,h)pyren, 9,10-Dibromanthracen, Riboflavin, 3,9-Dibromperylen, 3,10-Dibromperylen und 3,10-Dibrom-4,9-perylendicarboxylat.

5. Verfahren nach Anspruch 1 oder Anspruch 2, dadurch gekennzeichnet, daß der Absorber/Emitter ein Phosphor ist.

6. Verfahren nach Anspruch 5, dadurch gekennzeichnet, daß das Phosphor ausgewählt ist aus 2,3-Butadion, 1-[Carboxyphenyl]-1,2-pentandion und 1-Phenyl-1,2-propandion.

7. Verfahren nach einem der vorhergehenden Ansprüche, dadurch gekennzeichnet, daß der Chemilumineszenzkatalysator ausgewählt ist aus Peroxidase, baktierieller Luciferase und Leuchtkäferluciferase.

8. Bausatz zur Herstellung einer Reagenzlösung zur Prüfung großer Antigene nach dem Verfahren von Anspruch 1, dadurch gekennzeichnet, daß er aus

(i) einem mit einem Absorber/Emitter verbundenen Antikörper,

(ii) einem mit einem Chemilumineszenzkatalysator verbundenen Antikörper und

(iii) einem Chemilumineszenzreagenz besteht, das aus einem mit einem Chemilumineszenzcofaktorgeneratorkatalysator verbundenen Antikörper besteht, der zur Bildung eines Chemilumineszenzcofaktors befähigt ist, welcher in Gegenwart des Chemilumineszenzkatalysators ein Lichtsignal hervorrufen kann.

9. Bausatz nach Anspruch 8, dadurch gekennzeichnet, daß er einen Chemilumineszenzcofaktorfängerkatalysator enthält.

10. Bausatz nach Anspruch 8, dadurch gekennzeichnet, daß er mit Peroxidase verbundene Antikörper, mit Riboflavin verbundene Antikörper, Luminol, mit Glucoseoxidase verbundene Antikörper, Glucose und Catalase, einen mit einem Emitter verbundenen Antikörper und Chemilumineszenzreagenzien enthält, die erforderlich sind, um in Gegenwart des mit dem Chemilumineszenzkatalysator verbundenen Antigens ein chemilumineszierendes Lichtsignal hervorzurufen.

**Revendications**

1. Méthode pour titrer des antigènes ayant de nombreux sites de liaison, caractérisée en ce qu'elle comprend les étapes suivantes:

(a) on introduit un échantillon dans une solution réactive comprenant (i) un anticorps conjugé à un absorbeur/émetteur, (ii) un anticorps conjugué à un catalyseur de chimiluminescence et (iii) un réactif de chimiluminescence comprenant un anticorps conjugué à un catalyseur générateur de cofacteur de chimiluminescence, capable de produire un cofacteur de chimiluminescence qui peut induire une réponse lumineuse en présence du catalyseur de chimiluminescence; et

(b) on détecte toute réponse lumineuse émise par l'anticorps conjugué à l'absorbeur/émetter.

2. Méthode suivant la revendication 1, caractérisée en ce que le réactif comprend un catalyseur balayeur de cofacteur de chimiluminescence.

3. Méthode suivant la revendication 1 ou 2, caractérisée en ce que l'absorbeur/émetteur est un fluorophore.

4. Méthode suivant la revendication 3, caractérisée en ce que le fluorophore est choisi parmi l'uroporphyrine, la tétracarboxyphénylporphyrine, le pérylène, l'anthracène, le 7-méthylbibenzo(a,h)pyrène, le 9,10-dibromoanthracène, la riboflavine, le 3,9-dibromopérylène, le 3,10-dibromopérylène, et un 3,10-dibromo-4,9-pérylènedicarboxylate.

5. Méthode suivant la revendication 1 ou 2, caractérisée en ce que l'absorbeur/émetteur est un phosphore.

6. Méthode suivant la revendication 5, caractérisée en ce que le phosphore est choisi parmi la 2,3-butadione, la 1-[carboxyphényl]-1,2-pentanedione et la 1-phényl-1,2-propanedione.

7. Méthode suivant l'une quelconque des revendications précédentes, caractérisée en ce que le catalyseur de chimiluminescence est choisi parmi la peroxydase, la luciférase bactérienne et la luciférase de luciole.

8. Kit pour préparer une solution réactive pour le titrage de gros antigènes suivant la méthode de la revendication 1, caractérisé en ce qu'il comprend (i) un anticorps conjugué à un absorbeur/émetteur, (ii) un anticorps conjugué à un catalyseur de chimiluminescence et (iii) un réactif de chimiluminescence comprenant un anticorps conjugué à un catalyseur générateur de cofacteur de chimiluminescence, capable de produire un cofacteur de chimiluminescence qui peut induire une réponse lumineuse en présence du catalyseur de chimiluminescence.

9. Kit suivant la revendication 8, caractérisé en ce qu'il comprend un catalyseur balayeur de cofacteur de chimiluminescence.

10. Kit suivant la revendication 9, caractérisé en ce qu'il comprend des anticorps conjugués à la peroxydase, des anticorps conjugués à la riboflavine, du luminol, des anticorps conjugués à la glucose oxydase, du glucose et de la catalase.

Fig.1.

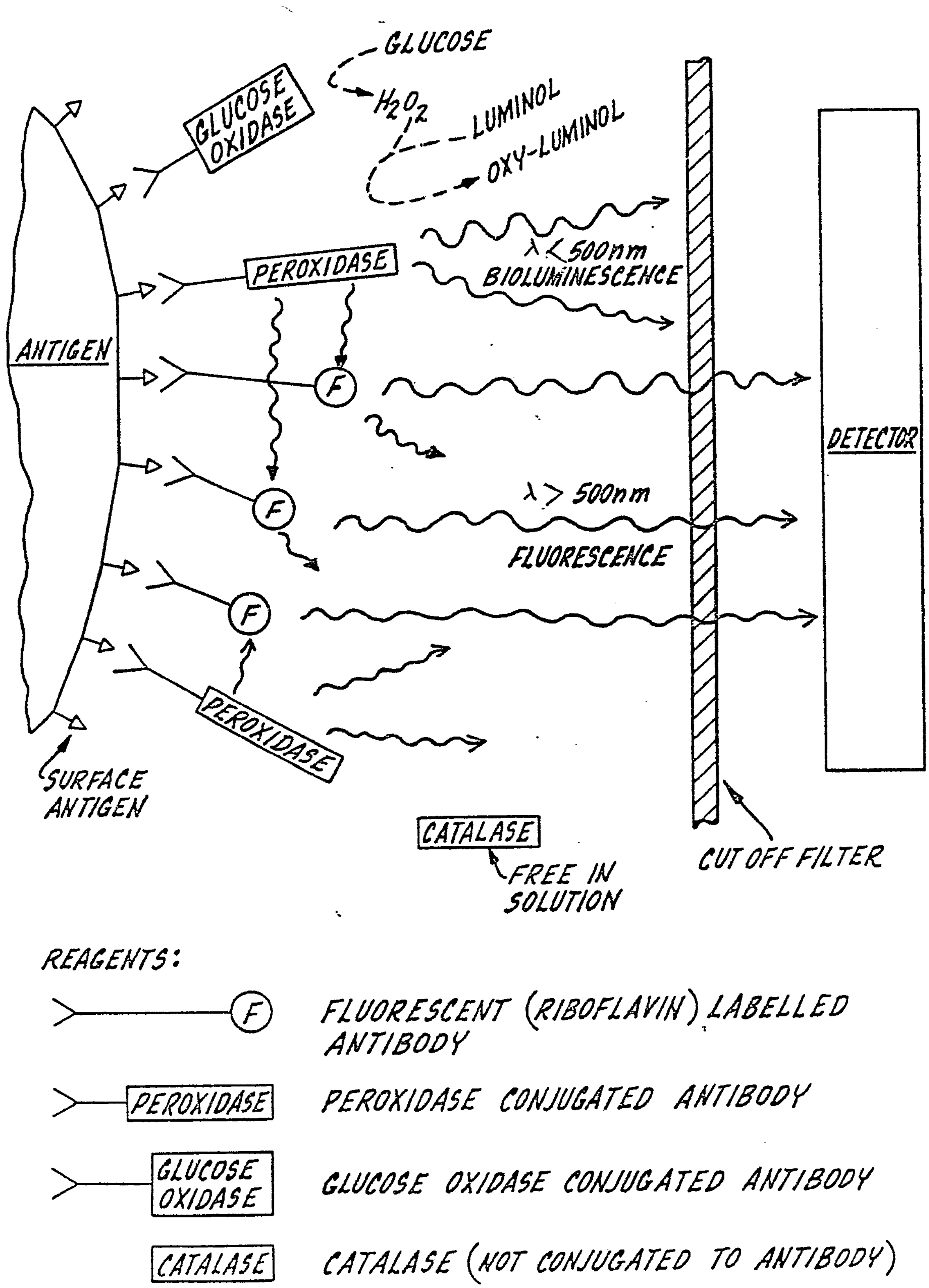
GLUCOSE
H2O2
LUMINOL
OXY-LUMINOL
GLUCOSE OXIDASE
PEROXIDASE
λ < 500nm
BIOLUMINESCENCE
ANTIGEN
F
DETECTOR
λ > 500nm
FLUORESCENCE
PEROXIDASE
SURFACE ANTIGEN
CATALASE
FREE IN SOLUTION
CUT OFF FILTER
REAGENTS:
F
FLUORESCENT (RIBOFLAVIN) LABELLED ANTIBODY
PEROXIDASE
PEROXIDASE CONJUGATED ANTIBODY
GLUCOSE OXIDASE
GLUCOSE OXIDASE CONJUGATED ANTIBODY
CATALASE
CATALASE (NOT CONJUGATED TO ANTIBODY)

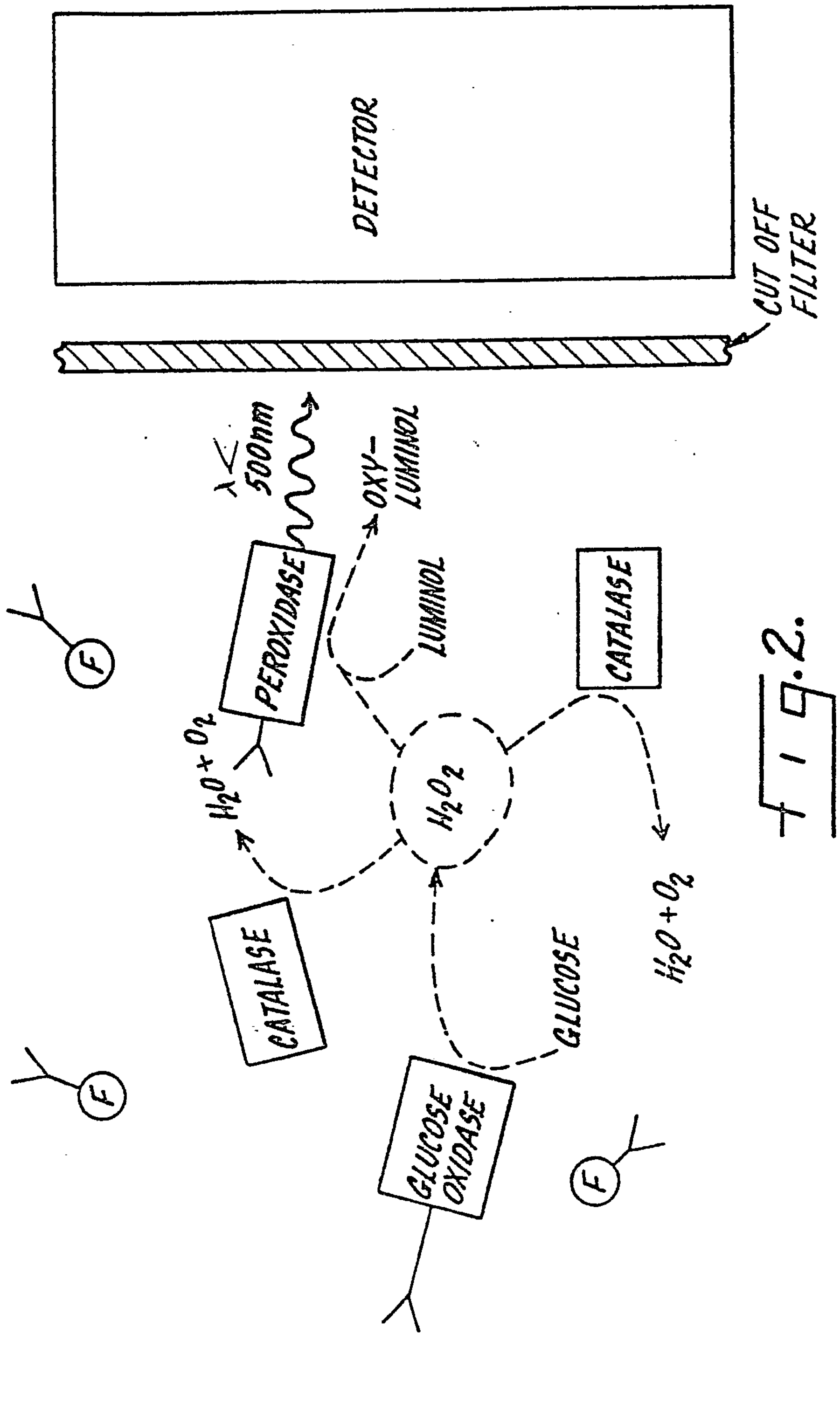
DETECTOR
CUT OFF FILTER
λ < 500nm
OXY-LUMINOL
LUMINOL
PEROXIDASE
$H_2O + O_2$
$H_2O_2$
CATALASE
CATALASE
$H_2O + O_2$
GLUCOSE
GLUCOSE
GLUCOSE OXIDASE
F
F
F

Fig. 2.